# EUROPEAN PATENT SPECIFICATION

(11) **EP 1 035 132 B1**
(45) Date of publication and mention of the grant of the patent: **14.05.2008**
(21) Application number: 98941848.8
(22) Date of filing: 11.09.1998
(51) Int. Cl.: C07K 16/18, C07K 16/28, C12N 5/20, A61K 39/395, C12P 21/08, C12N 15/06

(54) **MONOCLONAL ANTIBODY INDUCING APOPTOSIS**
MONOKLONALE ANTIKÖRPER ZUR APOPTOSIS-INDUZIERUNG
ANTICORPS MONOCLONAL INDUISANT L'APOPTOSE

(30) Priority: 11.09.1997 JP 26485397
(43) Date of publication of application: 13.09.2000
(73) Proprietor: CHUGAI SEIYAKU KABUSHIKI KAISHA, Tokyo, 115-8543 (JP)
(72) Inventor: FUKUSHIMA, Naoshi Chugai Seiyaku Kabushiki Kaisha, Gotemba-shi Shizuoka 412-8513 (JP); UNO, Shinsuke Chugai Seiyaku Kabushiki Kaisha, Gotemba-shi Shizuoka 412-8513 (JP)
(74) Representative: HOFFMANN EITLE
(86) International application number: PCT/JP1998/004118
(87) International publication number: WO 1999/012973

(56) References cited:
- EP-A- 0 721 015
- WO-A-97/32601
- WO-A1-97/32601
- LINDBERG F P ET AL: "MOLECULAR CLONING OF INTEGRIN-ASSOCIATED PROTEIN: AN IMMUNOGLOBULINFAMILY MEMBER WITH MULTIPLE MEMBRANE-SPANNING DOMAINS IMPLICATED INALPHAVBETA3-DEPENDENT LIGAND BINDING" JOURNAL OF CELL BIOLOGY, ROCKEFELLER UNIVERSITY PRESS, NEW YORK, US, US, vol. 123, no. 2, October 1993 (1993-10), pages 485-496, XP002929109 ISSN: 0021-9525
- MATEO V ET AL: "Induction of apoptosis in B-cells from chronic lymphocyte leukemia (B-CLLs) by CD47" FASEB JOURNAL, FED. OF AMERICAN SOC. FOR EXPERIMENTAL BIOLOGY, BETHESDA, MD, US, vol. 12, no. 5, 20 March 1998 (1998-03-20), page A1082, XP002105096 ISSN: 0892-6638
- FUKUSHIMA N ET AL: "APOPTOSIS OF BONE MARROW CELLS VIA INTEGRIN ASSOCIATED PROTEIN BY THE NOVEL MONOCLONAL ANTIBODY" BLOOD, W.B.SAUNDERS COMPAGNY, ORLANDO, FL, US, vol. 94, no. 10SUPPL01PT01, 15 November 1999 (1999-11-15), page 479A, XP001037645 ISSN: 0006-4971
- JOURNAL OF CELL SCIENCE, Vol. 108, No. 11, (1995), MARTINA I. REINHOLD et al., "In Vivo Expression of Alternatively Spliced Forms of Integrin-Associated Protein (CD47)", p. 3419-3425, XP002917383
- AVENT N. ET AL: 'Monoclonal antibodies that recognize different membrane proteins that are deficient in Thnull human erythrocytes' BIOCHEM. J. vol. 251, 1988, pages 499 - 505
- REINHOLD M.I. ET AL: 'In vivo expression of alternatively spliced forms of integrin-associated protein CD47' JOURNAL OF CELL SCIENCE vol. 108, no. 11, 1995, CAMBRIDGE UNIVERSITY PRESS, LONDON, GB, pages 3419 - 3425, XP002917383
- BROWN E.: 'Integrin-associated protein: a 50-Kd plasma membrane antigen physically and functionally associated with integrins' THE JOURNAL OF CELL BIOLOGY vol. 111, 1990, pages 2785 - 2794

## Description

### TECHNICAL FIELD

This invention relates to novel monoclonal antibodies designated MABL-1 or MABL-2 produced by FERM-BP-6100 or FERM BP-6101 having the property of inducing apoptosis of nucleated blood cells with Integrin Associated Protein (IAP), as well as to their fragments, peptides and low molecular weight compounds, and to hybridomas that produce the monoclonal antibodies. The novel antibodies are useful as therapeutic agents for myeloid leukemia and lymphoid leukemia.

### BACKGROUND ART

Granulocyte colony-stimulating factors, such as recombinant granulocyte colony-stimulating factor (rG-CSF), have been known in the prior art as humoral factors that stimulate differentiation and proliferation of granulocytes. Reports based on *in vivo* experiments with mice have shown that administration of rG-CSF results in not only accelerated myelopoiesis in bone marrow but also notable extramedullary hemopoiesis in the spleen, and proliferation of all hemopoietic precursor cells, including hemopoietic stem cells, in the spleen. The mechanism of such extramedullary hemopoiesis in the spleen has been believed that stimulation by rG-CSF alters the hemopoietic microenvironment of the spleen and promotes the hemopoiesis supporting ability thereof, thus inducing hemopoiesis.

In order to elucidate the hemopoietic function in the spleen, the present inventors have previously focused on stromal cells of the spleen following repeated administration of rG-CSF. The inventors have made efforts to examine how the hemopoietic function is promoted by rG-CSF via stromal cells, and have established a hemopoietic stromal cell line (CF-1 cells) from mouse spleen by repeated administration of rG-CSF. The inventors have studied the hemopoiesis-supporting ability of the hemopoietic stromal cells and confirmed the colony-stimulating activity *in vitro* and the hemopoietic stem cell-supporting ability *in vivo* [Blood, 80, 1914 (1992)].

However, while one cell line of the splenic stromal cells has been established (CF-1 cells) and its cytological characteristics have been studied, specific antibodies that recognize the surface antigens of these cells have never been prepared, nor have their characteristics been elucidated yet in any way.

### DISCLOSURE OF INVENTION

In light of the aforementioned findings relating to splenic stromal cells and the results of prior research, the present inventors have earnestly made further research aiming at developing specific antibodies that can recognize the splenic stromal cells, made efforts to prepare monoclonal antibodies using the aforementioned splenic stromal cell line as a sensitizing antigen, and finally succeeded in obtaining novel monoclonal antibodies.

The inventors have further studied the properties of the monoclonal antibodies obtained as above and found that the monoclonal antibodies have the property of inducing myeloid cell apoptosis. These monoclonal antibodies have been designated "BMAP-1 antibody", which will be hereinafter referred to as such.

The inventors have also examined the antigen recognized by BMAP-1 antibody and found that it is mouse Integrin Associated Protein (mouse IAP) (GenBank, Accession Number Z25524) by direct expression cloning.

The action of BMAP-1 antibodies has been studied using recombinant cells into which the gene for mouse IAP had been introduced. Specifically, the mouse IAP gene was introduced into mouse Jurkat cells, which did not express mouse IAP, by a conventional method to create a mouse IAP-expressing cell line (recombinant Jurkat cells), and the action of BMAP-1 antibody on the mouse IAP-expressing cells has been investigated by MTS assay and DNA fragmentation by using flow cytometry (Japanese Patent Application No. HEI 9-67499).

It has been expected upon these findings that monoclonal antibodies for the antigen of human Integrin Associated Protein (hereinafter referred to as human IAP; amino acid sequence and base sequence described in J. Cell Biol., 123, 485-496, 1993; see also Journal of Cell Science, 108, 3419-3425, 1995) should have an effect of inducing apoptosis of nucleated blood cells that express this antigen (myeloid cells and lymphocytes), and the present inventors have made efforts to prepare monoclonal antibodies for the antigen of human Integrin Associated Protein and succeeded in obtaining monoclonal antibodies that induce apoptosis of human nucleated blood cells expressing this antigen.

In other words, it is an object of this invention to provide novel monoclonal antibodies having the property of inducing apoptosis of nucleated blood cells (myeloid cells and lymphocytes) with human Integrin Associated Protein (human IAP), and fragments thereof, as well as hybridomas that produce the monoclonal antibodies.

These novel monoclonal antibodies are useful as therapeutic agents for myeloid leukemia and lymphoid leukemia.

The reported functions of Integrin Associated Protein are the action of binding with the β chain of integrin αVβ3 to support binding between αVβ3 and its ligand vitronectin (J. Cell. Biol., 123, 485-496 (1993)), that of inducing inflow of Ca²⁺ into the vascular endothelium upon adhesion of neutrophils with the vascular endothelium (J. Biol. Chem., 268, 19931-19934 (1993)), and that of supporting migration of neutrophils through the vascular endothelium (Proc. Natl. Acad. Sci. USA, 92, 3978-3982 (1995)), but no reports have been published on its function relating to apoptosis of nucleated blood cells.

EP A-0721015 describes an antibody BMAP-1, which neither binds to human IAP nor induces apoptosis of nucleated blood cells having human IAP.

N. Fukushima et al. describe the apoptosis of bone marrow cells via an integrin associated protein by a monoclonal antibody in BLOOD, vol. 94, 1999, 479A.

N. Avent et al. describe monoclonal antibodies that recognize different membrane proteins that are deficient in Thₙᵤₗₗ human erythrocytes in Biochem. J., 1988, 251, 499-505.

M.I. Reinhold et al. describe the in vivo expression of alternatively spliced forms of integrin - associated protein (CD47) in the Journal of cell science, 108, 3419-2425, 1995.

E. Brown describes an integrin-associated protein: a 50-kD plasma membrane antigen physically and functionally associated with integrins in the journal of cell biology, 111, Dec 1990, 2785-2794.

All documents do not describe MABL-1 and MABL-2.

The monoclonal antibodies of the invention are antibodies that specifically recognize human Integrin Associated Protein. They therefore exhibit a function of distinguishing and identifying human Integrin Associated Protein.

In addition, the monoclonal antibodies of the invention are antibodies that exhibit the property of inducing apoptosis of nucleated blood cells (myeloid cells and lymphocytes) with human Integrin Associated Protein. Apoptosis is a phenomenon in which nuclear chromatin DNA is cleaved into nucleosome units (known as a "ladder formation"), resulting in death of the cell and which is also referred to as cell suicide.

Monoclonal antibodies hitherto known to have the property of inducing apoptosis of nucleated blood cells (myeloid cells and lymphocytes) include anti-Fas antibody (Cell, 66; 233-243, 1991), anti-CD43 antibody (Blood, 86, 502-511, 1995) and anti-HLA Class Iα1 Domain antibody (Blood, 90, 726-735, 1997), but the property of inducing apoptosis of nucleated blood cells by the Integrin Associated Protein-recognizing antibodies of this invention has never been known. The monoclonal antibodies of the invention are therefore defined as encompassing any monoclonal antibody capable of specifically recognizing Integrin Associated Protein and having the property of inducing apoptosis of nucleated blood cells (myeloid cells and lymphocytes) with Integrin Associated Protein.

The antibodies of the invention are not limited only to those that induce apoptosis of all nucleated blood cells. They also include those that induce apoptosis of at least one type of nucleated blood cells. Specifically, it is sufficient in the case of myeloid leukemia to induce apoptosis of at least myeloid cells.

More specifically, this invention provides monoclonal antibodies that induce apoptosis of nucleated blood cells having Integrin Associated Protein (IAP).

The invention further provides fragments, peptides and low molecular compounds of monoclonal antibodies that induce apoptosis of nucleated blood cells having Integrin Associated Protein (IAP).

The invention still further provides hybridomas that produce the monoclonal antibodies.

The invention still further provides an antileukemic agent that contains a substance that binds to IAP and promotes the action of IAP to induce apoptosis of nucleated blood cells.

The invention still further provides an antileukemic agent characterized in that the substance is a monoclonal antibody.

The invention still further provides an antileukemic agent characterized in that the substance is a fragment, a peptide or a low molecular compound of the monoclonal antibodies.

### BRIEF DESCRIPTION OF DRAWINGS

Fig. 1 is an electrophoresis pattern showing a band for human IAP amplified by PCR using cDNA prepared from mRNA of HL-60 cell line. From left are shown a molecular weight marker (M), human IAP (1) and β-actin (2).
Fig. 2 is a graph showing the level of expression of human IAP by L1210 cells that have expressed human IAP, using anti-CD47 antibody. The peak represents L1210 cells transfected with only pCOS1 gene as a control.
Fig. 3 is another graph showing the level of expression of human IAP by L1210 cells that have expressed human IAP, using anti-CD47 antibody. The peak shows that human IAP expression has definitely increased in L1210 cells transfected with the human IAP gene.
Fig. 4 is a graph showing antibody titers in immunized mice. The left peak represents intact L1210 cells. The right peak represents L1210 cells transfected with human IAP, showing that the serum of the mouse subjected to cell fusion clearly recognizes human IAP.
Fig. 5 is a bar graph showing the results of a growth inhibition experiment (Jurkat cells) using a hybridoma culture supernatant.
Fig. 6 is a bar graph showing the results of a growth inhibition experiment (ARH77 cells) using a hybridoma culture supernatant.
Fig. 7 is a graph showing the apoptosis-inducing effect on Jurkat cells by a culture supernatant (as analyzed by PI staining), which is the result for an 8G2 culture supernatant used as a control. R1 indicates the percentage (%) of apoptosis, which is 7.43%.
Fig. 8 is a graph showing the apoptosis-inducing effect on Jurkat cells by a culture supernatant (as analyzed by PI staining), which is the result for 7D2-E3. R1 indicates the percentage (%) of apoptosis, which is 9.84%.
Fig. 9 is a graph showing the apoptosis-inducing effect on Jurkat cells by a culture supernatant (as analyzed by PI staining), which is the result for 11C8. R1 indicates the percentage (%) of apoptosis, which is 15.32%.
Fig. 10 is a graph showing the apoptosis-inducing effect on HL-60 cells by a culture supernatant (as analyzed by PI staining), which is the result for an 8G2 culture supernatant used as a control. M1 indicates the percentage (%) of apoptosis, which is 6.94%.
Fig. 11 is a graph showing the apoptosis-inducing effect on HL-60 cells by a culture supernatant (as analyzed by PI staining), which is the result for 11C8. M1 indicates the percentage (%) of apoptosis, which is 12.16%.
Fig. 12A is a monochrome photomicrograph showing the result of apoptosis analysis (TUNEL method) in a coculturing system with KM-102 and HL-60 cells, using 9C5 culture supernatant as a control. The apoptotic cells are stained black or brown. The nuclear staining was accomplished with Methyl Green, and the magnification is 100x.
Fig. 12B is a color photomicrograph showing the result of apoptosis analysis (TUNEL method) in a coculturing system with KM-102 and HL-60 cells, using 9C5 culture supernatant as a control. The apoptotic cells are stained black or brown. The nuclear staining was accomplished with Methyl Green, and the magnification is 100x.
Fig. 13A is a monochrome photomicrograph showing the result of apoptosis analysis (TUNEL method) in a coculturing system with KM-102 and HL-60 cells, using 11C8 culture supernatant. More TUNEL-positive cells are seen than in Fig. 12. The apoptotic cells are stained black or brown. The nuclear staining was accomplished with Methyl Green, and the magnification is 100x.
Fig. 13B is a color photomicrograph showing the result of apoptosis analysis (TUNEL method) in a coculturing system with KM-102 and HL-60 cells, using 11C8 culture supernatant. More TUNEL-positive cells are seen than in Fig. 12. The apoptotic cells are stained black or brown. The nuclear staining was accomplished with Methyl Green, and the magnification is 100x.
Fig. 14 is an electrophoresis pattern showing the results of SDS-PAGE analysis of IgG purified from hybridoma lines 7D2-E3 and 11C8. Shown are molecular weight markers (M, M'), mouse IgG (authentic sample) under non-reducing conditions (1), 7D2-E3 (2), 11C8 (3), mouse IgG (authentic sample) under reducing conditions (4), 7D2-E3 (5) and 11C8 (6).
Fig. 15 shows the results of analysis of CD47 expression by flow cytometry, using HL-60 cells.
Fig. 16 shows the results of analysis of CD47 expression by flow cytometry, using Jurkat cells.
Fig. 17 shows results for mIgG (10 µg/ml) as a control to demonstrate its apoptosis-inducing effect on L1210 cells transfected with the human IAP gene (L1210-hIAP) (incubation for 72 hours).
Fig. 18 shows the apoptosis-inducing effect of MABL-1 (10 µg/ml) on L1210 cells transfected with the human IAP gene (incubation for 72 hours).
Fig. 19 shows the apoptosis-inducing effect of MABL-2 (10 µg/ml) on L1210 cells transfected with the human IAP gene (incubation for 72 hours).
Fig. 20 shows results for mIgG (10 µg/ml) as a control to demonstrate its apoptosis-inducing effect on Jurkat cells (incubation for 48 hours).
Fig. 21 shows the apoptosis-inducing effect of MABL-1 (10 µg/ml) on Jurkat cells (incubation for 48 hours).
Fig. 22 shows the apoptosis-inducing effect of MABL-2 (10 µg/ml) on Jurkat cells (incubation for 48 hours).
Fig. 23 shows results for mIgG (10 µg/ml) as a control to demonstrate its apoptosis-inducing effect on L1210 cells transfected with the human IAP gene introduced therein (L1210-hIAP) (incubation for 72 hours).
Fig. 24 shows the apoptosis-inducing effect of MABL-2 Fab fragments (10 µg/ml) on L1210 cells transfected with the human IAP gene.
Fig. 25 is an SDS electrophoresis pattern for MABL-2 antibody Fab fragments.
Fig. 26 shows a notably extended survival period upon treatment with MABL-2.
Fig. 27 shows the results of ELISA for Example 5(2).
Fig. 28 shows a notably extended survival period upon treatment with MABL-2 F(ab')2 fragments.
Fig. 29 is an SDS electrophoresis pattern for MABL-1 antibody and MABL-2 antibody F(ab')2 fragments.
Fig. 30 shows that human IgG levels of mouse serum were decreased significantly in the groups treated with MABL-1 and MABL-2, which indicates anti-tumor effects of these antibodies.

### BEST MODE FOR CARRYING OUT THE INVENTION

### Preparation of Monoclonal Antibody

The monoclonal antibodies of this invention can generally be prepared in the following manner. That is, monoclonal antibodies of the invention may be obtained, for example, by using human Integrin Associated Protein as the sensitizing antigen, immunizing animals with the antigen by an immunization method known in the art, performing cell fusion by a cell fusion method known in the art and cloning by a cloning method known in the art.

More specifically, a preferable method of preparing monoclonal antibodies of the invention is, for example, a method wherein recombinant cells of the mouse leukemia cell line L1210 that express human Integrin Associated Protein are used as the sensitizing antigen, plasma cells (immunocytes) of a mammal immunized with the sensitizing antigen are fused with myeloma cells of mammals such as mice, the resulting fused cells (hybridomas) are cloned, the clones producing the antibodies of the invention that recognize the aforementioned cell line are selected from the resulting clones and cultured, and the target antibodies are obtained.

The above method is merely one possible example of the invention and, for example, the sensitizing antigen is not limited to the aforementioned L1210 recombinant cells but may also be human Integrin Associated Protein (IAP) itself, or human IAP in soluble form; the target monoclonal antibodies that induce apoptosis of nucleated blood cells (myeloid cells and lymphocytes) can be prepared in the same manner as in the L1210 recombinant cells mentioned above.

The phage display method may also be used to prepare a target monoclonal antibody from a cDNA library for the antibody.

The mammals to be immunized with the sensitizing antigen in the method of preparing the monoclonal antibodies are not particularly limited, but they are preferably selected in consideration of their compatibility with the myeloma cells used for cell fusion, and mice, rats, hamsters and the like are general suitable.

The immunization is preferably accomplished by a standard method. For example, the human Integrin Associated Protein-expressing L1210 recombinant cells are administered to the animal by intraperitoneal injection or the like. More specifically, an appropriate dilution or suspension with PBS or physiological saline is preferably administered to the animal a few times at 10-day intervals. The immunocytes used are preferably spleen cells extracted after the final administration of the cells.

The mammalian myeloma cells used as the parent cells for fusion with the immunocytes may be any of various cell lines known in the art, for example, P3 (P3X63Ag8.653) [J. Immunol., 123, 1548 (1978)], P3-U1 [Current Topics in Microbiology and Immunology, 81, 1-7 (1978)], NS-1 [Eur. J. Immunol., 6, 511-519 (1976)], MPC-11 [Cell, 8, 405-415 (1976)], Sp2/0-Agl4 [Nature, 276, 269-270 (1978)], FO [J. Immunol. Meth., 35, 1-21 (1980)], S194 [J. Exp. Med., 148, 313-323 (1978)] and R210 [Nature, 277, 131-133 (1979)].

The cell fusion between the immunocytes and myeloma cells may be performed basically according to a conventional method, such as the method of Milstein et al. [Methods Enzymol., 73, 3-46 (1981)].

More specifically, the cell fusion is carried out, for example, in a common nutrient medium in the presence of a fusion promoter. For example, the fusion promoter used may be polyethylene glycol (PEG), Sendai virus (HVJ) or the like, and, if desired, an adjuvant such as dimethyl sulfoxide may also be added appropriately in order to increase fusion efficiency. The immunocytes are used preferably in the amount of 1-10 times as much as myeloma cells. The medium used for the cell fusion may be, for example, RPMI-1640 medium, MEM medium and the like, which are suitable for growth of myeloma cell lines, or other media commonly used for such cell culturing, and it may also be used in combination with a serum supplement such as fetal bovine serum (FBS).

The cell fusion is carried out by thoroughly mixing prescribed amounts of the immunocytes and myeloma cells in the medium, adding a solution of PEG preheated to about 37°C, the PEG having an average molecular weight of approximately 1,000-6,000, for example, to the medium usually at a concentration of about 30-60% (W/V), and mixing. A suitable medium is then successively added, and the supernatant obtained by centrifugation is removed. This procedure is repeated to produce the target hybridomas.

The hybridomas are selected by culturing in a common selection medium, such as HAT medium (a medium containing hypoxanthine, aminopterin and thymidine). Culturing in the HAT medium is continued for a sufficient time to allow death of all the cells other than the target hybridomas (all the non-fused cells), which is usually from a few days to a few weeks. The usual limiting dilution method is then employed for screening and monocloning of hybridomas producing the target antibodies.

The hybridomas prepared in this manner that produce the monoclonal antibodies of the invention may be subcultured in common medium, and may be placed in long-term storage in liquid nitrogen.

In order to obtain the monoclonal antibodies of the invention from the hybridomas, any suitable methods may be employed, such as a method wherein the hybridomas may be cultured according to standard methods and the antibodies may be obtained from the culture supernatants; or alternatively, a method wherein the hybridomas may be administered to a compatible mammal for proliferation and then the antibodies may be obtained from the ascites fluid thereof. The former method is suitable for obtaining highly pure antibodies, while the latter method is more suited for mass production of antibodies.

The antibodies obtained by the aforementioned methods can be highly purified by utilizing standard purification methods such as salting-out, gel filtration, affinity chromatography, or the like.

### Monoclonal Antibody Fragments

The monoclonal antibodies of this invention may be the complete antibodies described above, or fragments thereof. That is, they may be any fragments of a monoclonal antibody of the invention that specifically recognize human Integrin Associated Protein and induce apoptosis of nucleated blood cells (myeloid cells and lymphocytes) having human Integrin Associated Protein. Such fragments include Fab, F(ab')₂, Fab', etc. These fragments can be prepared by digestion with an enzyme such as papain, pepsin, ficin or the like. The properties of the obtained fragments can be confirmed in the same manner as described above.

### Peptides and Low Molecular Compounds Having the Same Function as the Monoclonal Antibodies

The monoclonal antibodies described above, which recognize human Integrin Associated Protein and induce apoptosis of nucleated blood cells, also encompass peptides and low molecular compounds that likewise recognize IAP and induce apoptosis of nucleated blood cells.

### Properties of Monoclonal Antibodies of the Invention

As specifically described in the following Examples, the monoclonal antibodies of the invention specifically recognize human Integrin Associated Protein.

The monoclonal antibodies of the invention also induce apoptosis of nucleated blood cells (myeloid cells and lymphocytes) with human Integrin Associated Protein.

These properties can be utilized to obtain useful therapeutic agents in the field of treatment for myeloid leukemia and lymphoid leukemia.

Thus, it will be readily appreciated that the construction of specific systems involving the use of the monoclonal antibodies of the invention, as antibodies to specifically recognize an antigen that causes apoptosis of nucleated blood cells, for distinction and identification of the antigens, or the use of the unique properties of the monoclonal antibodies as therapeutic agents for myeloid leukemia and lymphoid leukemia, as well as any modifications and applications of the system, are also within the scope of this invention insofar as they can be carried out by applying standard methods that are obvious to those skilled in the art.

### Antileukemic Agents

An antileukemic agent according to this invention is based on the fact that the action of IAP is promoted by binding of an antibody of the invention. While there are no particular limitations on the dose of the antibody of the invention, it is preferably in the range of 5 µg to 500 mg/kg.

### EXAMPLES

This invention will now be explained in greater detail by way of the following examples.

### Example 1 (Monoclonal Antibody Preparation)

### 1) Sensitizing antigen and immunization method

Antigen sensitization was accomplished using a recombinant cell line as the sensitizing antigen, which was the L1210 cells transfected with human IAP gene and highly expressed the product. L1210 is obtained from the DBA mouse-derived leukemia cell line (ATCC No. CCL-219, J. Natl. Cancer Inst. 10:179-192, 1949).

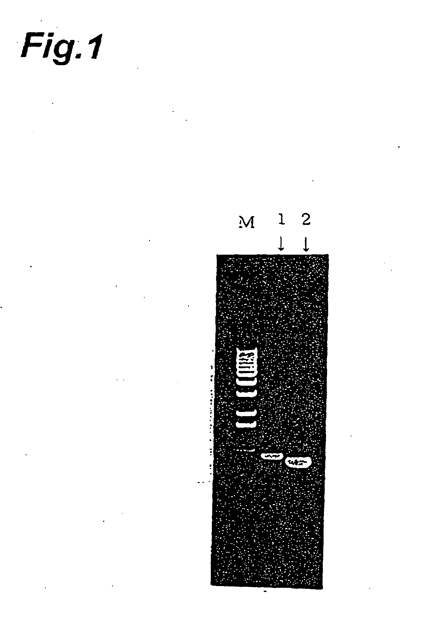

The human IAP gene was amplified by PCR using a primer with a human IAP-specific sequence (sense primer: GCAAGCTTATGTGGCCCCTGGTAGCG, antisense primer: GCGGCCGCTCAGTTATTCCTAGGAGG) and cDNA prepared from mRNA of HL-60 cell line (Clontech laboratories, Inc.) as the template (Fig. 1).

The PCR product was subcloned into a cloning vector pGEM-T (Promega Corporation) and used to transform *E*. *coli* JM109 (Takara Shuzo Co., Ltd.), and after confirming the nucleotide sequence of the insert DNA with a DNA sequencer (373A DNA Sequencer, available from ABI), it was subcloned with an expression vector pCOS1.

Expression vector pCOS1 is a derivative of pEF-BOS (Nucleic Acids Research, 18, 5322, 1990), and it is a vector obtained by subcloning the neomycin resistant gene using human elongation factor-1α as a promoter/enhancer. This human IAP-subcloned expression vector was used for gene introduction into L1210 cell line with DMRIE-C (GIBCO/BRL), selection was performed with Geneticin (final concentration: 1 mg/ml, available from GIBCO/BRL), and the gene-introduced L1210 cells were cloned by the limiting dilution method.

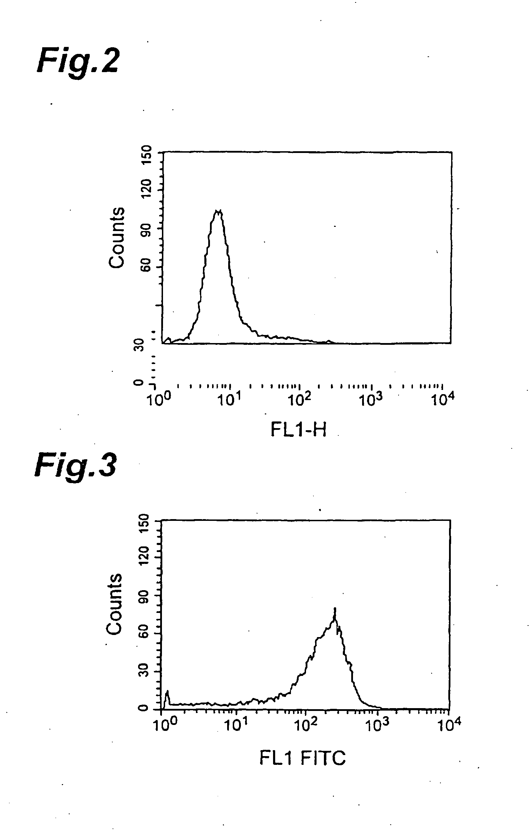

The antigen expression of the obtained clones was examined using human IAP-recognizing anti-CD47 antibody (PharMingen), and the clones with high levels of expression were selected as antigen-sensitized cells (Figs. 2, 3). For culturing of the recombinant L1210 cells, 10% fetal bovine serum (FBS, available from Moregate Inc.) and Iscove's-Modified Dulbecco's Medium (IMDM) (GIBCO/BRL) were used as the medium, and the cells were subcultured in a 5% CO₂ incubator at a temperature of 37°C.

The immunized animals used were DBA/2 mice (bred by Charles River, Japan), which were of the same strain as the L1210 cells. The human Integrin Associated Protein (IAP) gene-transfected L1210 cells, used for antigen sensitization, were incubated for about 30 min with mitomycin C (Kyowa Hakko Kogyo Co., Ltd.) at a concentration of 200 µg/ml, and after suspending growth of the cells, mitomycin C was thoroughly washed off prior to suspension in PBS.

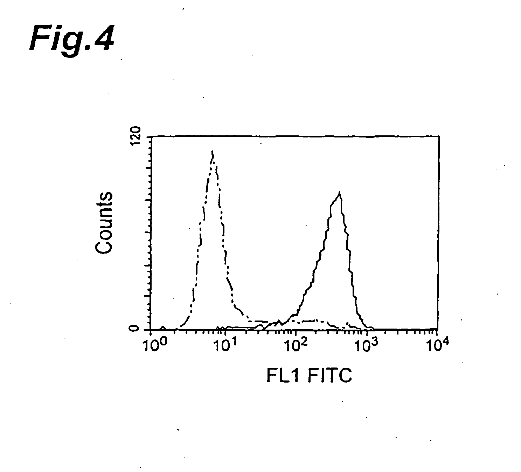

The cells were intraperitoneally injected into the mice three times at intervals of about 10 days, at approximately 5 × 10⁶ cells each time. After the third immunization, blood was taken from the eye socket, the serum was diluted 50-fold with PBS containing 1% BSA, and binding between the diluted serum and the recombinant L1210 cells used for antigen sensitization was confirmed with a FACScan (Becton Dickinson and Company) (Fig. 4); the mouse having the best antiserum activity was subjected to a booster immunization with intraperitoneal injection of 1 × 10⁷ cells 5 days after the fourth immunization. Four days after the final immunization, the mouse was sacrificed and the spleen extracted.

### (2) Cell fusion

After thinly slicing the spleen extracted from the mouse, the dissociated spleen cells were centrifuged and then suspended in IMDM medium, allowed to float, and thoroughly rinsed. Separately, the mouse myeloma cell line P3-U1 [Current Topics in Microbiology and Immunology, 81, 1-7 (1978)] was cultured in IMDM medium containing 10% fetal bovine serum (FBS, available from Moregate Inc.), and after rinsing similarly with the IMDM medium, the 1 × 10⁷ cells were placed in a centrifuge tube in admixture with 5 × 10⁷ cells of the spleen cells and subjected to cell fusion according to a standard method [Clin. Exp. Immunol., 42, 458-462 (1980)], using polyethylene glycol 4000 (Nakarai Chemical Co., Ltd.).

The resulting fused cells were then suspended in IMDM medium containing 10% FBS and a fused cell growth stimulating agent (BM-Condimed H1, available from Boehringer Mannheim Biochemicals) and dispensed into a 96-well plate for culturing at 37°C in a 5% CO₂ incubator. On the following day, the cells were placed in the HAT selection medium and then the 10% FBS/IMDM medium containing the growth-stimulating agent, and culturing was continued to sustain growth.

In order to examine the effect of the culture supernatant of these fused cells on leukemia cell lines, the medium for fused cells was replaced with IMDM medium containing 10% FBS, and culturing was continued to sustain growth.

### (3) Screening

The following screening was performed using the culture supernatant of the aforementioned fused cells.

### [1] Primary screening

Cells of a mouse spleen stromal cell line (CF-1 cells) transfected with the human Integrin Associated Protein (IAP) gene (recombinant cells into which the same plasmid was subcloned as the plasmid used to prepare the human IAP-expressing L1210 cells used for antigen sensitization) were seeded in a 96-well plate at 1 × 10⁴ cells per well and cultured overnight, and then fixed with 2% PLP (periodate-lysine-paraformaldehyde) to prepare an ELISA plate. After rinsing, the plate was subjected to blocking for 1 h at room temperature using a 1% BSA solution, and after further rinsing, 50 µl of the culture supernatant of each hybridoma was added for incubation at room temperature for one hour.

After rinsing, anti-mouse IgG+A+M (H+L) (Zymed Laboratories Inc.) labeled with alkaline phosphatase was added prior to incubation at room temperature for 1 h. After rinsing, SIGMA 104 substrate (Sigma-Aldrich Corporation) was added to provide a final concentration of 1 mg/ml, incubation was continued at room temperature, and the specific activity was measured with a microplate reader (Model 3550, available from BioRad Laboratories Inc.).

As a result, appearance of hybridomas was confirmed in 2089 wells among the hybridomas seeded in 2880 wells, with 187 wells being positive in the primary screening. 50 µl each of Mouse IgG1 as a negative control and anti-human CD47 antibody (BD PharMingen) as a positive control were added at a concentration of 3 µg/ml, respectively, prior to incubation at room temperature for 1 h.

### [2] secondary screening

The clones judged as positive in the primary screening were subjected to an ELISA system using human Integrin Associated Protein (IAP)-expressing CF-1 cells, where the negative control was CF-1 cells transfected with only the expression vector pCOS1, in order to screen whether the antibodies produced by the hybridomas would specifically recognize human IAP.

As a result, the positive was confirmed for 21 of the 187 wells found to be positive in the primary screening. Table 1 shows the specific binding of human IAP with 7D2 and 11C8 as representative examples among these, in terms of the absorbance in ELISA. (Table 1) ELISA analysis of specific binding of hybridoma culture supernatants with human IAP

**Table 1**

| <Raw data> | PBS | αhCD47 3 µg/ml | 7D2 | 11C8 |
|---|---|---|---|---|
| CF1-pCOS1 | 0.185 | 0.160 | 0.189 | 0.149 |
| CFl-hIAP-55-8 | 0.192 | 0.456 | 0.568 | 0.812 |
| <Subtracted> | PBS | αhCD47 3 µg/ml | 7D2 | 11C8 |
| Specific binding | 0.007 | 0.296 | 0.379 | 0.663 |

### [3] Tertiary screening

The clones judged to be positive in the secondary screening were subjected to a growth inhibition test using Jurkat cells (human T cell lymphoma line) and ARH77 cells (human myeloma cell line). 100 µl of the Jurkat cells at 5 × 10³ cells per well and the ARH77 cells at 1 × 10⁴ cells per well were seeded in each well of a 96-well plate, and 5 or 10 µl of culture supernatant of the hybridoma clones were added to the cell suspensions. After culturing for about 2 days, the cell numbers were measured by MTS assay. As a control, 5 or 10 µl each of IMDM medium containing 10% FBS and culture supernatants of clones that were negative in the primary screening (8G2 and 9C5) were added.

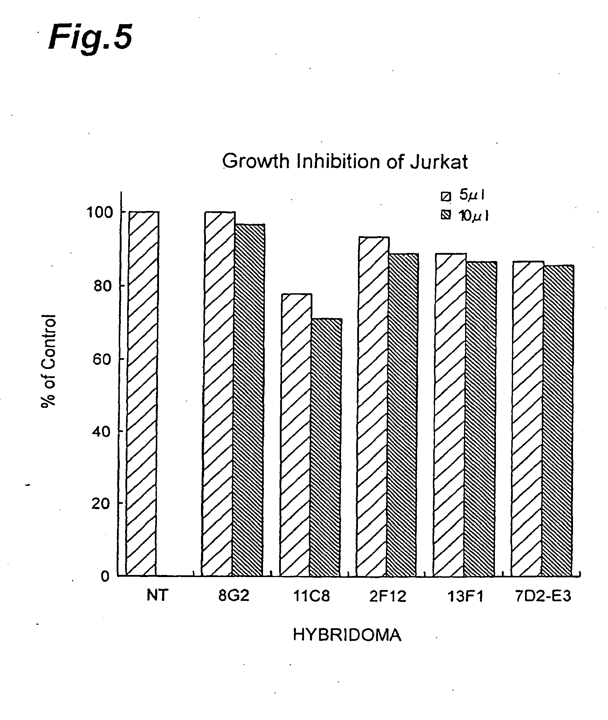

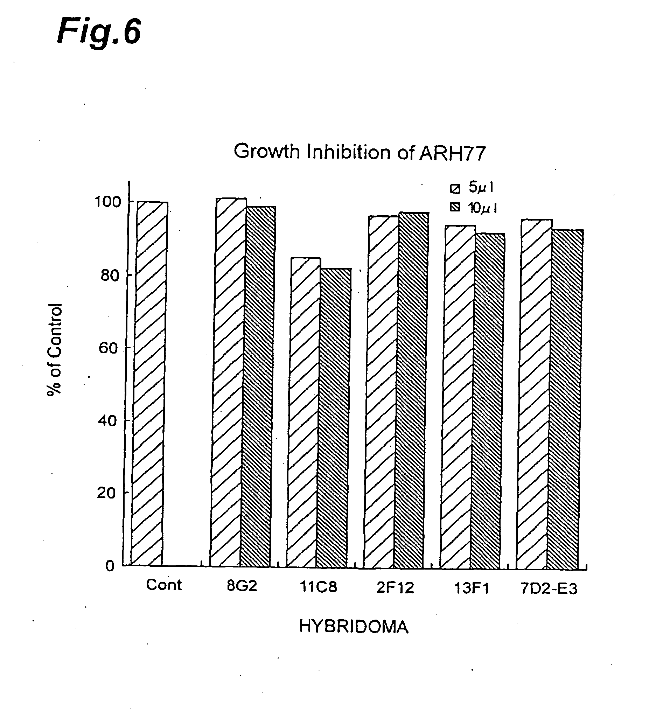

Figs. 5 and 6 show the results of the growth inhibition effect of four representative clones, 11C8, 7D2-E3 (subclone of 7D2), 13F1 and 2F12.

### (4) Antibody properties

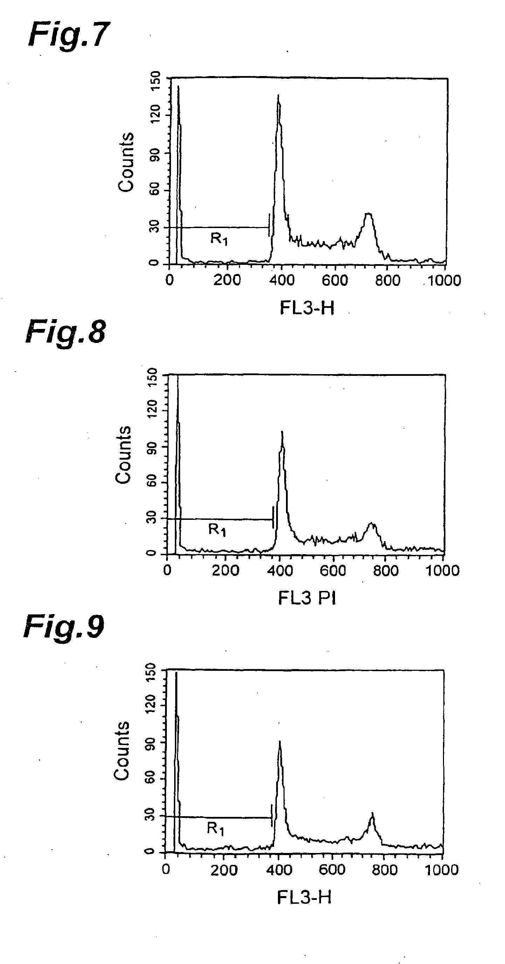

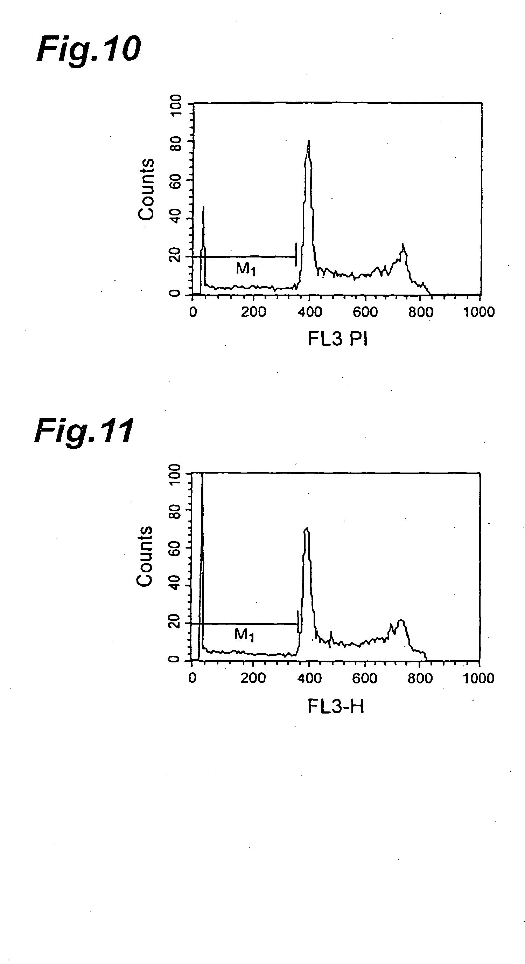

[1] The immunoglobulin types of the culture supernatants of 11C8, 7D2-E3, 13F1 and 2F12 were examined using an ELISA system.
   Specifically, human Integrin Associated Protein (IAP)-expressing CF-1 cells were seeded into a 96-well plate to prepare an ELISA plate, and then 50 µl of each culture supernatant was added, alkaline phosphatase-labeled anti-mouse IgG antibody (Zymed Laboratories Inc.) or anti-mouse IgM antibody (Biosource Intl., Inc.) were reacted therewith as secondary antibodies, and the activity was measured with a microplate reader. As a result, 11C8 and 7D2-E3 were confirmed to be IgG, while 13F1 and 2F12 were confirmed to be IgM.
[2] The DNA fragmentation of the two clones 11C8 and 7D2-E3 among the four clones described above was analyzed by flow cytometry (FACScan, available from Becton, Dickinson and Company) using Jurkat cells and HL-60 cells. The Jurkat cells were used for 11C8 and 7D2-E3, and the HL-60 cells were used for 11C8.
   The Jurkat cells and HL-60 cells were seeded in a 12-well plate at 4 × 10⁴ cells per well/2 ml, respectively, and 200 µl of the culture supernatants of 7D2-E3 and 11C8 were added. The cells were cultured for 2 days, and measured. As a control, 8G2 culture supernatant was added in an equal volume. The cells were recovered from the culturing plate and a cell pellet was fixed under 200 x g for 60 minutes at 4°C in 2 ml of chilled 70% ethanol. The cells were then centrifuged, rinsed in 1 ml of PBS and resuspended in 0.5 ml of PBS. To a 0.5 ml sample of the cells, 0.5 ml of RNAse (Type I-A, Sigma-Aldrich Corporation, St. Louis, MO, USA; 1 mg/ml in PBS) was added, and these were mixed with a 1 ml propidium iodide solution (PI, Sigma, 100 µg/ml in PBS). The mixed cells were incubated for 60 min in a darkroom at 37°C, and then kept in the darkroom at 4°C and measured by flow cytometry.
   As shown in Figs. 7-9 and 10-11, the culture supernatants of 7D2-E3 and 11C8 increase a proportion of apoptosis cells of Jurkat cells and the culture supernatant of 11C8 increases a proportion of apoptosis cells of HL-60 cells, respectively.
[3] The culture supernatants of 11C8 were used in a coculturing system with HL-60 cells using a feeder layer of cells of the human myeloid stromal cell line KM102, to determine whether these culture supernatants induce apoptosis of HL-60 cells.

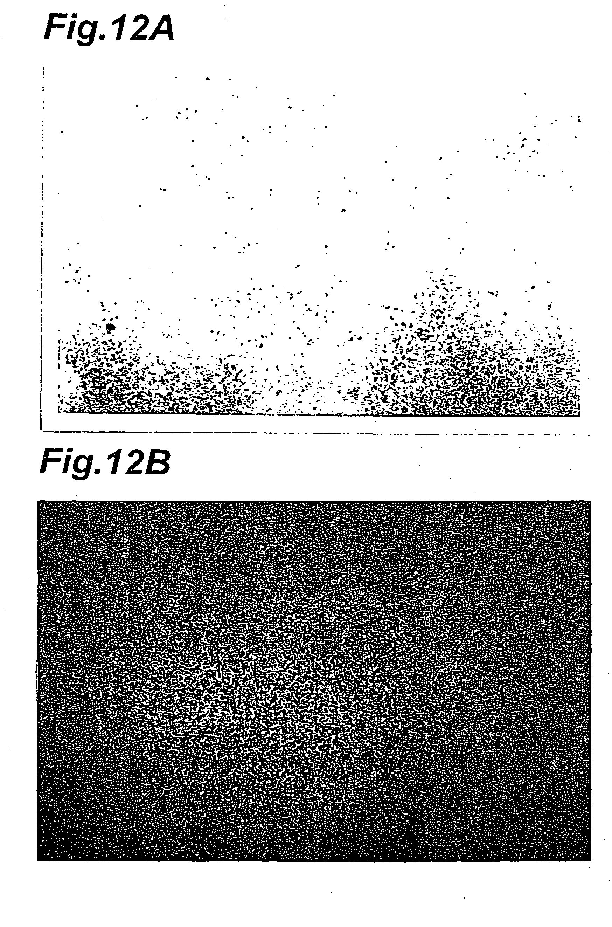

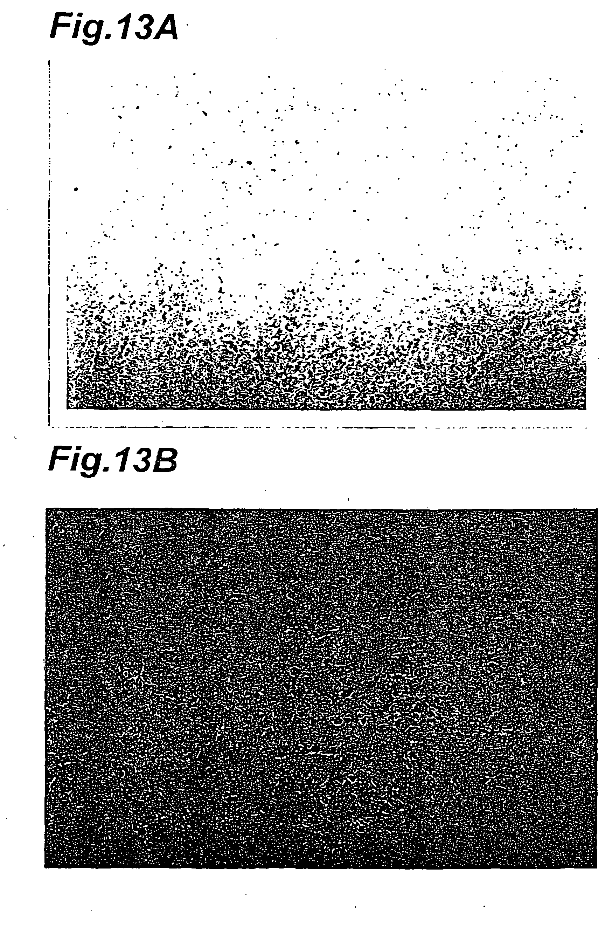

Specifically, KM102 cells were seeded in a 2-well Lab-Tek Chamber Slide (Nalge Nunc Intl. Corporation) and brought to a sub-confluent state, 1 × 10⁵ cells of HL-60 cells were seeded thereon and cultured for about one day, and then the non-attached HL-60 cells were removed. The aforementioned culture supernatants were simultaneously added to provide a final concentration of 10% and the cells were cultured for 2 days. After culturing, the cells were fixed with 10% formalin and the apoptosis-induced HL-60 cells were detected by the TUNEL method (ApopTag Plus available from Oncor Inc.). As shown in Figs. 12 and 13, the culture supernatant of 11C8 more increases apoptosis cells of HL-60 cells than the culture supernatant of 9C5 does, which is the culture supernatant of the human IAP non-reacting hybridoma clone used as the control.

### (5) Antibody purification

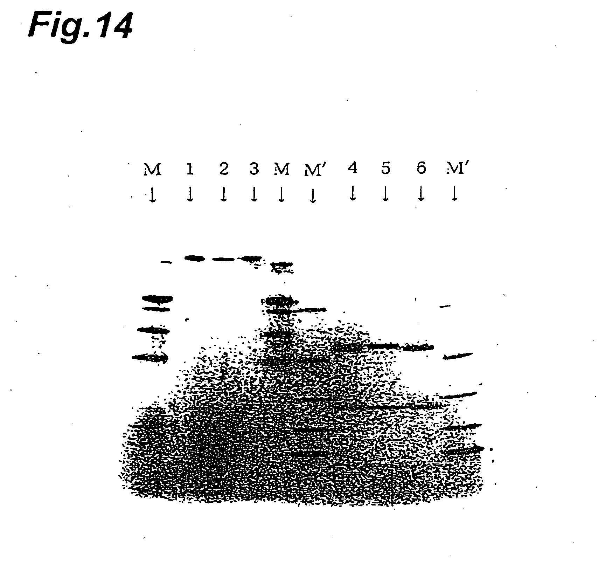

For purification of the antibodies produced by hybridomas, the cell lines of the IgG-producing clones 7D2-E3 and 11C8 among the above hybridoma lines were intraperitoneally injected into pristane-administered BALB/c/AnNCrj mice (male, available from Charles River, Japan) according to a standard method. After several weeks, the ascites fluid produced was taken and the antibodies were separated and purified by standard methods. Specifically, the antibodies were purified from the obtained ascites fluid by a Polos Protein A plastic column (Perceptive Biosystems Inc.) and dialyzed with PBS (Dulbecco Inc.), and bands were confirmed with SDS-PAGE analysis. As shown in Fig. 14, electrophoresis using an authentic sample of mouse IgG (Cappel Inc.) as a control confirmed bands for the IgG of clones 7D2-E3 and 11C8 at the same positions as the authentic sample mouse IgG, under both non-reducing conditions and reducing conditions.

In this example, the human Integrin Associated Protein (IAP)-expressing L1210 cells were used as the sensitizing antigen for illustrative purposes, but it is also possible to prepare monoclonal antibodies in the same manner using other human IAP-expressing cells or human IAP itself, and to prepare monoclonal antibodies from an antibody library using the phage display method; this invention is not limited to the aforementioned monoclonal antibodies but encompasses all monoclonal antibodies with properties similar thereto and all hybridomas that produce those monoclonal antibodies.

Furthermore, the invention of these monoclonal antibodies also includes humanized antibodies, human antibodies, chimeric antibodies, single-chain antibodies, primatized antibodies and antibody fragments obtained by digesting the antibodies with various enzymes (papain, pepsin, ficin, etc.).

The hybridomas producing the monoclonal anti-human Integrin Associated Protein (IAP) antibodies of the invention are novel fused cells created from DBA mice spleen cells and the mouse myeloma cell line P3-U1 as the parent cells; anti-IAP antibody (mouse hybridoma 11C8-F8 (subclone of 11C8), designated as "MABL-1") was deposited as FERM BP-6100 and anti-IAP antibody (mouse hybridoma 7D2-E3 (subclone of 7D2), designated as "MABL-2") as FERM BP-6101 on September 1, 1997 with the National Institute of Bioscience and Human Technology, Agency of Industrial Science and Technology, Ministry of International Trade and Industry, located at 1-3 Higashi 1-chome, Tsukuba-shi, Ibaraki-ken, Japan, as an authorized depository for public microorganisms.

### Example 2 (Subclass identification of MABL-1 and MABL-2 antibodies)

In order to identify the subclasses of MABL-1 and MABL-2 antibodies obtained above, 500 µl each of MABL-1 and MABL-2 adjusted to 100 ng/ml was spotted on an Isotyping Kit (Stratagene), by which MABL-1 was shown to be IgG1, κ and MABL-2 was shown to be IgG2a, κ.

### Example 3 (Human IAP-expressing human leukemia cells)

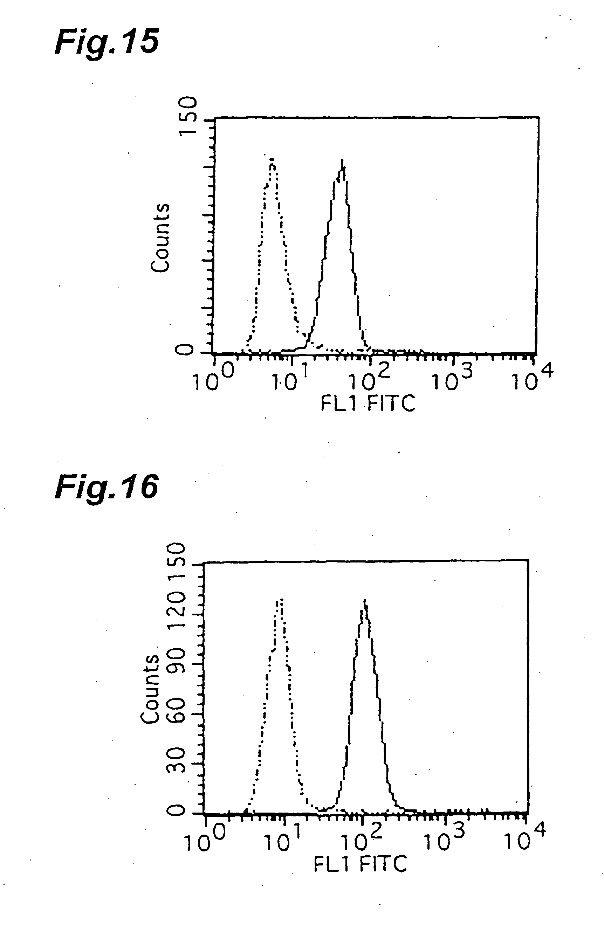

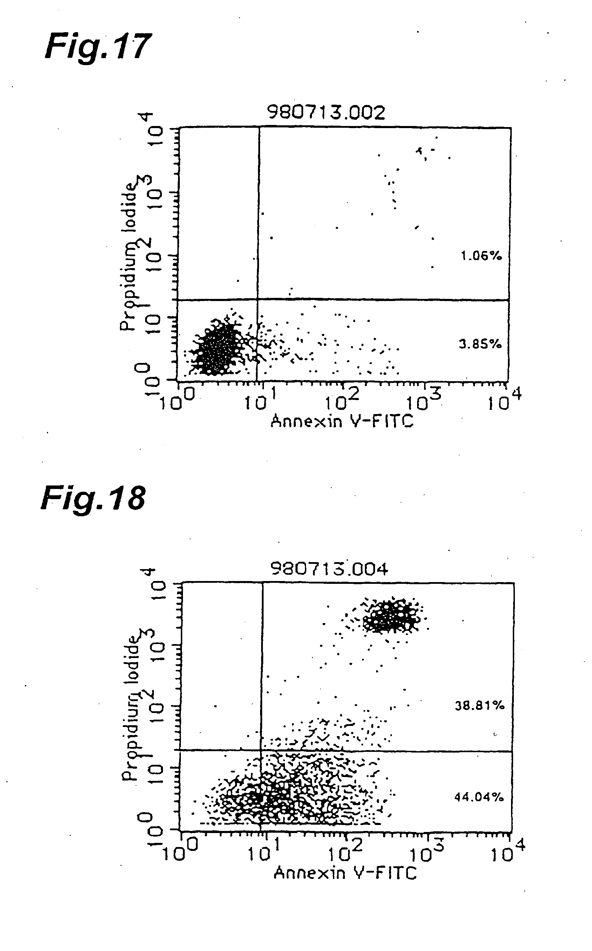

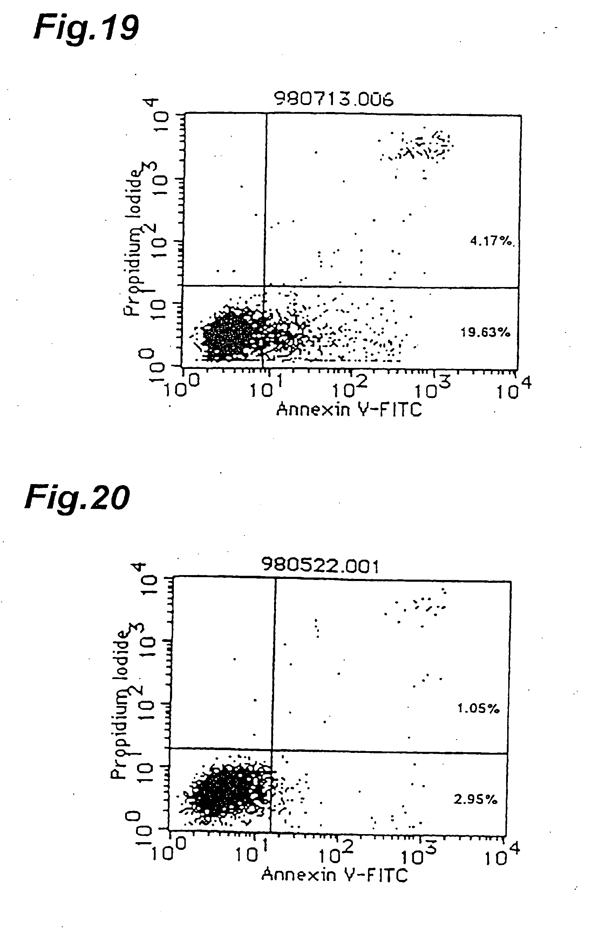

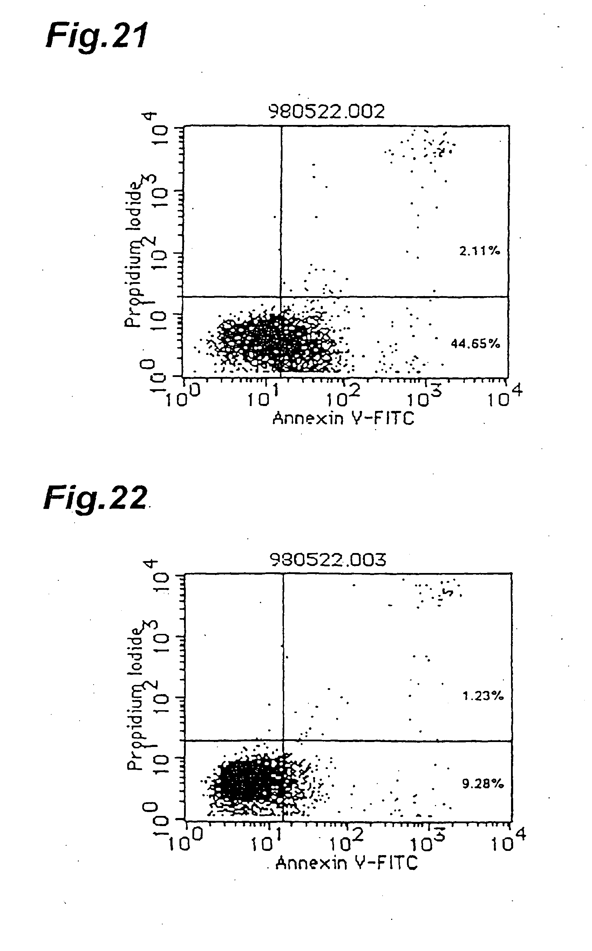

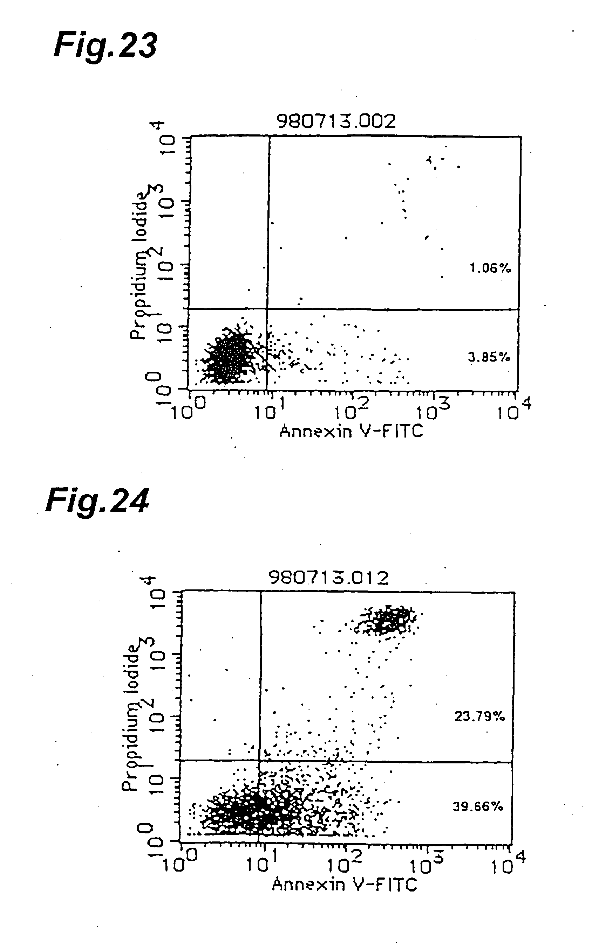

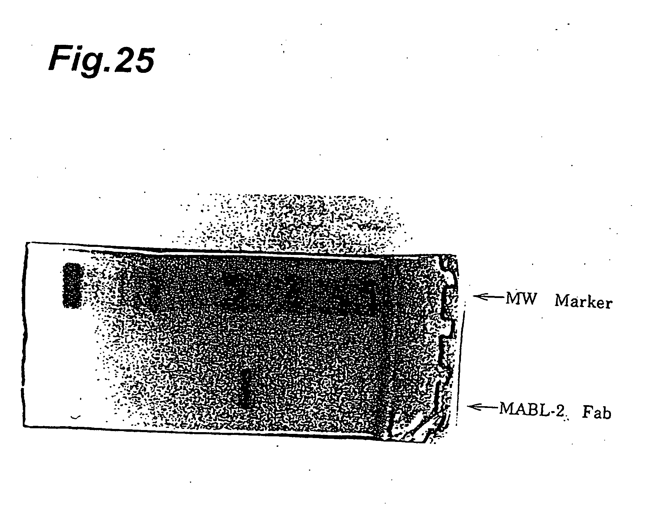

IAP expression in different human leukemia cell lines was detected by flow cytometry with human IAP-recognizing anti-CD47 antibody (a commercially available product). This antibody was used for the detection because human IAP is believed to be identical to CD47 (Biochem. J., 304, 525-530, 1994). The cell lines used were Jurkat and HL-60 cells (K562 cells, ARH77 cells, Raji cells and CMK cells). The cells were used at 2 × 10⁵ cells per sample, the anti-CD47 antibody was incubated with the cells at a final concentration of 5 µg/ml, and the secondary antibody used was FITC-labeled anti-mouse IgG antibody (Becton Dickinson and Company). Mouse IgGl antibody (Zymed Laboratories Inc.) was used as a control. The results of the flow cytometry as shown in Fig. 15 (HL-60) and Fig. 16 (Jurkat) confirmed that both cell lines expressed IAP. Example 4 (Apoptotic effect *in vitro*)
(1) The apoptosis-inducing activity of the MABL-1 and MABL-2 antibodies on L1210 cells transfected with human IAP gene, Jurkat cells and HL-60 cells were examined using Annexin-V (Boehringer Mannheim). The results of analysis with Annexin-V are shown in Figs. 17-22, wherein the dots in the lower left region indicate the live cells, those in the lower right region indicate apoptotic cells, and those in the upper right region indicate necrotic cells. The antibodies used were mouse IgG (Zymed Laboratories Inc.) as a control and MABL-1 and MABL-2 at 10 µg/ml, and after 4 × 10³ cells of L1210 cells transfected with the human IAP gene were incubated for 72 h and 6 × 10⁴ cells of the Jurkat cells were incubated for 48 h, they were analyzed with Annexin-V. Cell death was observed, as shown in Figs. 17-22. For the HL-60 cells, 10 µg/ml of MABL-1 was used, and analysis with Annexin-V at 1 × 10⁵ cells likewise revealed cell death.
(2) The apoptosis-inducing activity of MABL-2 antibody Fab fragments on L1210 cells transfected with human IAP gene was examined. Specifically, L1210 cells transfected with human IAP gene were cultured at 4 × 10³ cells, and MABL-2 antibody Fab fragments and mouse IgG as a control were used at a concentration of 10 µg/ml. The cells were incubated for 72 h and measured with Annexin-V. As a result, considerable cell death was observed (Figs. 23, 24). The MABL-2 antibody Fab fragments used for the experiment were obtained by digesting the antibody with papain (Pierce Laboratories, Inc.) and purifying it. The MABL-2 antibody Fab fragments were analyzed by SDS electrophoresis (Fig. 25).

### Example 5 (Investigation of apoptosis in vivo)

### (1) Drug efficacy of MABL-1 and MABL-2 (whole IgG)

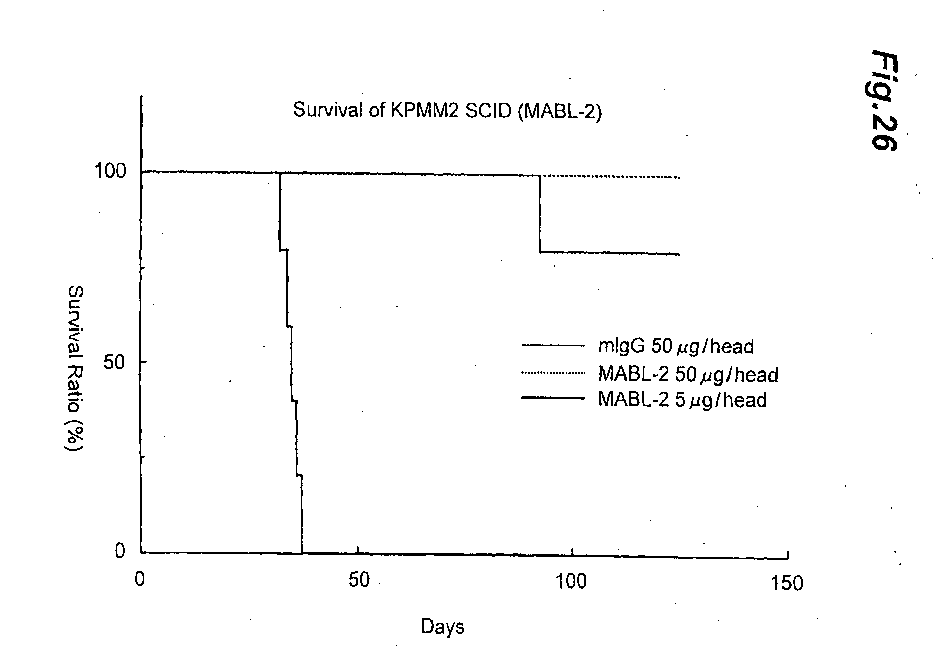

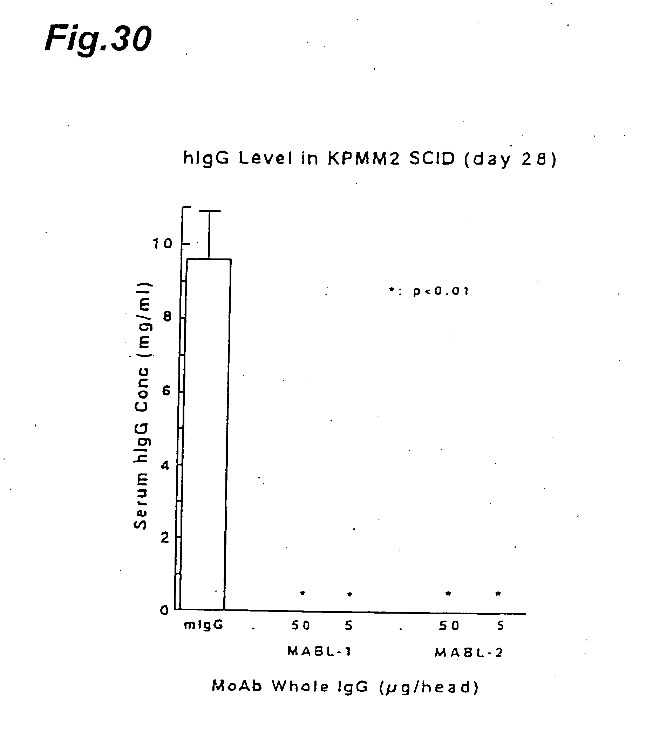

Human IAP-expressing KPMM2 cells (human myeloma cell line) were transplanted into SCID mice, and on the 10th day after transplantation, MABL-1 and MABL-2 (whole IgG) were administered by single intravenous injection in a dose each of 5 µg/head and 50 µg/head, respectively (n=5); on the 28th day after KPMM2 transplantation, the human IgG levels derived from KPMM2 were measured by ELISA, and the disappearance was confirmed. The survival period was also examined. The results showed marked suppression of blood levels of human IgG in the groups treated with MABL-1 and MABL-2, which represented the anti-tumor effect (Fig. 30). The survival period was also shown to be notably lengthened (Fig. 26).

### (2) Drug efficacy of MABL-1 and MABL-2 (F(ab')₂)

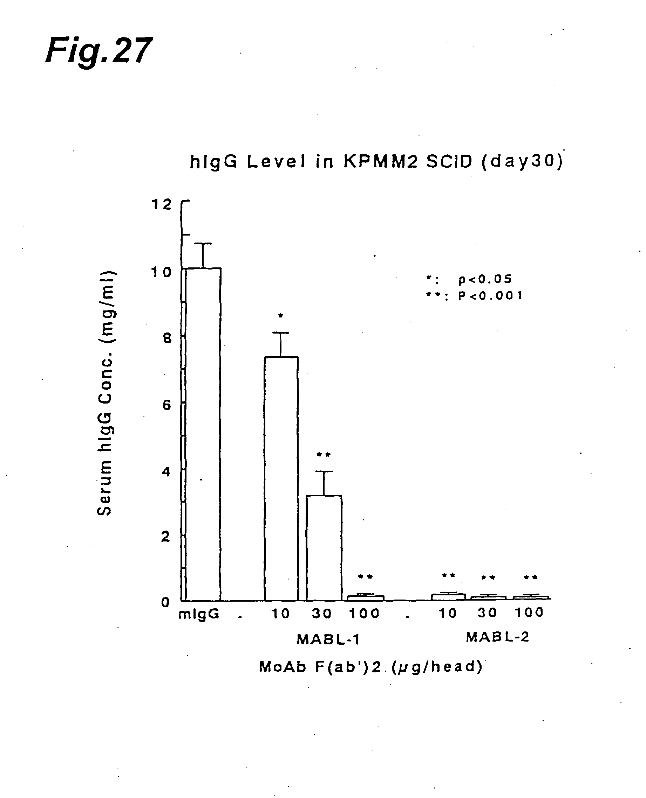

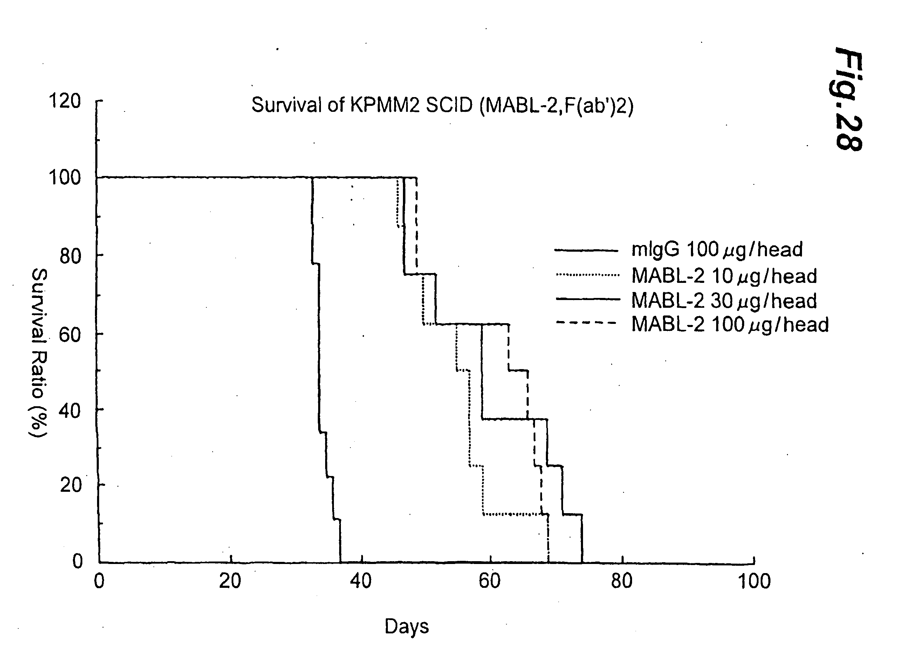

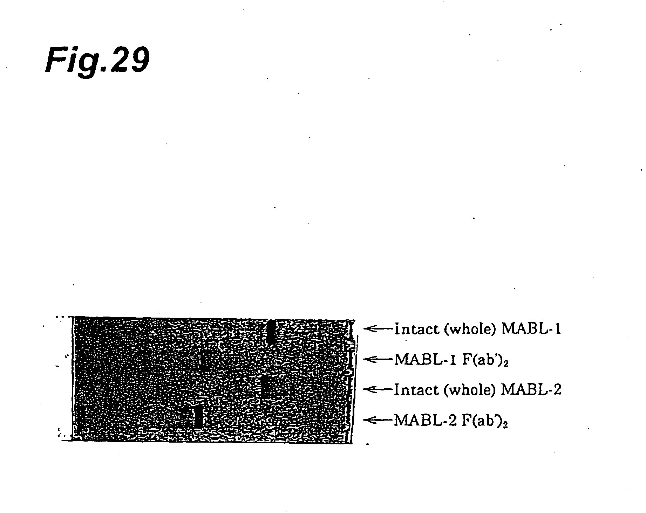

F(ab')₂ fragments prepared by digestion of the MABL-1 and MABL-2 antibodies with pepsin and purification with Protein A (Pierce laboratories, Inc.) were used to examine the anti-tumor effect except the cytotoxic effect via the Fc regions. Specifically, human IAP-expressing KPMM2 cells (human myeloma cell line) were transplanted into SCID mice, and MABL-1 and MABL-2 F(ab')₂ fragments were intravenously administered to the groups in a dose of 100 µg/head on the 6th and 10th days after transplanting, and to the groups in a dose each of 10 and 30 µg/head on the 6th, 8th and 10th days after transplantation, respectively; the human IgG levels derived from KPMM2 were measured by ELISA on the 30th day after transplantation (Fig. 27). The survival period was also examined up to 90 days after transplanting. As a result, a notable suppressing effect on human IgG levels in the blood was found in the groups treated with MABL-1 and MABL-2, which represented the anti-tumor effect. The survival period was also considerably lengthened (Fig. 28). Fig. 29 shows the SDS electrophoresis pattern for the F(ab')₂ fragments of MABL-1 antibody and MABL-2 antibody.

### INDUSTRIAL APPLICABILITY

The monoclonal antibodies of this invention are antibodies that specifically recognize human Integrin Associated Protein, and the antigens that induce apoptosis of nucleated blood cells having human Integrin Associated Protein. Accordingly, they are useful as antibodies that recognize human Integrin Associated Protein for its distinction and identification, while also having an action of inducing apoptosis of nucleated blood cells; these properties can be utilized to prepare useful therapeutic agents in the field of treatment for myeloid leukemia and lymphoid leukemia.

## Claims

1. An antibody designated as MABL-1 or MABL-2 produced by a hybridoma with the accession number FERM BP-6100 or FERM BP-6101, respectively, that binds to human Integrin Associated Protein (IAP) and induces apoptosis of nucleated blood cells having human IAP or an antibody that binds to the same antigenic determinant as the antibody designated,as MABL-1 or MABL-2 and induces apoptosis of nucleated blood cells having human IAP.

2. A fragment of an antibody of claim 1, said fragment binds to human IAP and induces apoptosis of nucleated blood cells having human IAP.

3. An antileukemic agent comprising an antibody of claim 1 or a fragment according to claim 2.

4. A hybridoma deposited with the accession number FERM BP-6100 or FERM BP-6101.

5. Use of an antibody of claim 1 or of a fragment according to claim 2 for the preparation of a medicament for the treatment of leukaemia.

6. Use according to claim 5, wherein the leukaemia is myeloid or lymphoid leukaemia.

7. An antibody against human IAP that is produced by immunising with cells expressing human IAP and is capable of inducing apoptosis of cells expressing human IAP.

8. An antibody of claim 7, wherein the antibody is IgG.

9. The antibody of claim 1 or the fragment of claim 2 for use in a method of treating leukaemia.

## Patentansprüche

1. Ein als MABL-1 oder MABL-2 bezeichneter Antikörper, hergestellt von einem Hybridom mit der Eingangsnummer FERM BP-6100 bzw. FERM BP-6101, der an menschliches Integrin-assoziiertes Protein (IAP) bindet und Apoptose von kernhaltigen Blutzellen, die menschliches IAP aufweisen, auslöst, oder ein Antikörper, der an dieselbe antigene Determinante wie der als MABL-1 oder MABL-2 bezeichnete Antikörper bindet und Apoptose von kernhaltigen Blutzellen mit menschlichem IAP auslöst.

2. Fragment des Antikörpers gemäß Anspruch 1, worin besagtes Fragment an menschliches IAP bindet und Apoptose von kernhaltigen Blutzellen mit menschlichem IAP auslöst.

3. Mittel gegen Leukämie, umfassend einen Antikörper gemäß Anspruch 1 oder ein Fragment gemäß Anspruch 2.

4. Hybridom, hinterlegt mit der Eingangsnummer FERM BP-6100 oder FERM BP-6101.

5. Verwendung eines Antikörpers gemäß Anspruch 1 oder eines Fragments gemäß Anspruch 2 für die Herstellung eines Medikaments zur Behandlung von Leukämie.

6. Verwendung gemäß Anspruch 5, worin die Leukämie myeloide oder lymphoide Leukämie ist.

7. Antikörper gegen menschliches IAP, welcher hergestellt wird durch Immunisieren mit Zellen, die menschliches IAP exprimieren und der in der Lage ist, Apoptose in Zellen, die menschliches IAP exprimieren, auszulösen.

8. Antikörper gemäß Anspruch 7, der ein IgG-Antikörper ist.

9. Antikörper gemäß Anspruch 1 oder das Fragment gemäß Anspruch 2 zur Verwendung in einem Verfahren zur Behandlung von Leukämie.

## Revendications

1. Anticorps désigné par MABL-1 ou MABL-2 produit par un hybridome, avec, respectivement, le nombre d'accès FERM BP-6100 ou FERM BP-6101, qui se lie à une Protéine Associée aux Intégrines (IAP) humaine et induit une apoptose de cellules sanguines nucléées ayant la IAP humaine ou un anticorps qui se lie au même déterminant antigénique comme l'anticorps désigné par MABL-1 ou MABL-2 et induit une apoptose de cellules sanguines nucléées ayant la IAP humaine.

2. Fragment d'un anticorps de la revendication 1, ledit fragment se lie à la IAP humaine et induit une apoptose de cellules sanguines nucléées ayant la IAP humaine.

3. Agent anti-leucémique comprenant un anticorps de la revendication 1 ou un fragment selon la revendication 2.

4. Hybridome déposé avec le nombre d'accès FERM BP-6100 ou FERM BP-6101.

5. Utilisation d'un anticorps de la revendication 1 ou d'un fragment selon la revendication 2 pour la préparation d'un médicament pour le traitement de la leucémie.

6. Utilisation selon la revendication 5, où la leucémie est une leucémie myéloïde ou lymphoïde.

7. Anticorps contre la IAP humaine qui est produit par immunisation avec des cellules exprimant la IAP humaine est capable d'induire une apoptose de cellules exprimant la IAP humaine.

8. Anticorps de la revendication 7, dans lequel l'anticorps est l'IgG.

9. Anticorps de la revendication 1 ou fragment de la revendication 2 à utiliser dans un procédé de traitement de la leucémie.


### SEQUENCE LISTING

<110> CHUGAI SEIYAKU KABUSHIKI KAISHA
<120> Monoclonal antibody inducing apoptosis
<130> 81932seq
<140> PCT/JP98/04118
   <141> 1998-09-11
<150> JP 9-264853
   <151> 1997-09-11
<160> 2
<210> 1
   <211> 26
   <212> DNA
   <213> Artificial Sequence
<220>
   <223> PCR primer
<400> 1
   GCAAGCTTAT GTGGCCCCTG GTAGCG 26
<210> 2
   <211> 26
   <212> DNA
   <213> Artificial Sequence
<220>
<223> PCR primer
<400> 2
   GCGGCCGCTC AGTTATTCCT AGGAGG 26